(19) Europäisches Patentamt
European Patent Office
Office européen des brevets

(11) Veröffentlichungsnummer: **0 190 993**
**B1**

# (12) EUROPÄISCHE PATENTSCHRIFT

(45) Veröffentlichungstag der Patentschrift: **08.11.89**

(51) Int. Cl.[4]: **C 07 C 147/02,** C 08 F 120/38

(21) Anmeldenummer: **86810042.1**

(22) Anmeldetag: **24.01.86**

## (54) Perfluoralkylsulfonoalkylacrylate und -methacrylate, ein Verfahren zu ihrer Herstellung und ihre Verwendung.

(30) Priorität: **30.01.85 US 697593**

(43) Veröffentlichungstag der Anmeldung:
**13.08.86 Patentblatt 86/33**

(45) Bekanntmachung des Hinweises auf die Patenterteilung:
**08.11.89 Patentblatt 89/45**

(84) Benannte Vertragsstaaten:
**BE CH DE FR GB IT LI NL**

(56) Entgegenhaltungen:
**US-A-3 655 732**

(73) Patentinhaber: **CIBA-GEIGY AG**
**Klybeckstrasse 141**
**CH-4002 Basel (CH)**

(72) Erfinder: **Kleiner, Eduard**
**Hemlock Hill Road**
**Pound Ridge New York 10576 (US)**
Erfinder: **Karydas, Athanasios**
**574 92nd Street**
**Brooklyn New York 11209 (US)**

**EP 0 190 993 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Beschreibung**

Die vorliegende Erfindung betrifft Perfluoralkylsulfongruppen enthaltend Acrylate und Methacrylate, Polymere davon und ihre Verwendung zur wasser-Und ölabweisenden und trockenen Schmutz abweisenden Ausrüstung von Cellulose oder natürlichen und synthetischen Polyamid- und Polyestermaterialien.

Die Erfindung betrifft ferner ein einfaches und wirtschaftliches Verfahren zur Herstellung der erwähnten Acrylate und Methacrylate.

Es sind bereits einige strukturell unterschiedliche Acrylate und Methacrylate bekannt, siehe zum Beispiel U.S. 2,642,416, 3,102,103, 3,282,906, 3,544,663, 3,655,732 und 4,060,681 und DE—A—2 052 579. Die Herstellung dieser Acrylate und Methacrylate umfasst aufwendige Mehrstufenverfahren, die mit einem unwertschaftlichen Verlust der teuren Perfluorverbindungen verbunden sind. Perfluoralkyloxyperfluoralkylacrylate und -methacrylate mit einer Thioethergruppe sind im US—Patent 3,763,116 beschrieben.

Die erfindungsgemässen Acrylate und Methacrylate entsprechen der Formel I

$$R_f(CH_2)_nSO_2(CH_2)_mO_2C{-}\overset{\displaystyle R}{\overset{|}{C}}{=}CH_2 \qquad (I),$$

worin

$R_f$ für Perfluoralkyl oder Perfluoralkoxy substituiertes Perfluoralkyl mit bis zu 18 C-Atomen steht,
R Wasserstoff oder Methyl bedeutet,
n 2 bis 20 und
m 2 bis 20 sind.

Die Gruppe $R_f$ kann biz zu 18, bevorzugt 3 bis 18, besonders 6 bis 16 und insbesondere 6 bis 12 C-Atome enthalten. Die Gruppe $R_f$ kann linear oder verzeigt sein. Die Gruppe $R_f$ kann vorteilhaft als Mischung von Perfluoralkylgruppen mit unterschiedlicher Anzahl an C-Atomen vorliegen.

Bei der Gruppe R handelt es sich bevorzugt um Methyl.

In Formel I sind n und m bevorzugt 2 bis 5. Insbesondere ist n 2 und m 2 bis 4. Insbesondere ist m gleich 3.

Die Verbindung der Formel I können in an sich bekannter Weise durch Veresterung oder Umesterung erhalten werden. Hierbei setzt man Verbindungen der Formel II

$$R_f(CH_2)_nSO_2(CH_2)_mOH \qquad (II),$$

mit Verbindungen der Formel III um,

$$CH_2{=}CR{-}CO{-}Z \qquad (III),$$

worin Z für Methoxy, Hydroxy oder Halogen, besonders Chlor, steht. Die Reaktion wird bevorzugt bei Temperaturen von 20 bis 100°C in Gegenwart eines organischen Lösungsmittels durchgeführt. Beispiele für Lösungsmittel sind Tetrahydrofuran, Dioxan oder Di-n-propylether. Zweckmässig wird die Reaktion in Gegenwart einer Base, z.B. tertiären Niederalkylaminen wie Trimethyl- oder Triethylamin durchgeführt, wenn Z in Formel III für Halogen steht, um den entstehenden Halogenwasserstoff zu binden. Man kann auch einen Ueberschuss der Verbindung der Formel III einsetzen, um die Bildung des gewünschten Esters zu vervollatständigen. Das gewünschte Reaktionsprodukt der Formel I kann mittels konventioneller Methoden isoliert werden, z.B. durch Destillation oder Ausfällung bei kristallinen Produkten. Die Produkte können nach der Isolierung durch Waschen mit Wasser oder Alkanolen wie z.B. Methanol und anschliessendem Trocknen weiter gereinigt werden.

Die Verbindungen der Formel II sind durch Oxidation eines Thioetheralkohols der Formel IV

$$R_f(CH_2)_nS(CH_2)_mOH \qquad (IV)$$

erhältlich.

Ein geeignetes Oxidationsmittel ist Wasserstoffperoxid in einem organischen sauren Medium, wie z.B. Essigsäure. Die Reaktion wird bei Temperaturen bis zu 100°C durchgeführt, bis der Thioetheralkohol in das entsprechende Sulfon überführt ist. Im allgemeinen begünstigen erhöhte Tempraturen, z.B. zwischen 50 und 100°C, die Bildung des Sulfons. Die Bildung des Sulfons wird auch durch Verwendung eines wesentlichen Ueberschusses von Wasserstoffperoxid begünstigt.

Die Alkohole der Formel IV sind bekannt oder können nach bekannten Verfahren in einfacher Weise hergestellt werden. Die Alkohole der Formel IV können beispielsweise durch Reaktion eines Merkaptans der Formel V

$$R_f(CH_2)_nSH \qquad (V),$$

mit entweder einem Halogenalkanol der Formel VI

$$X(CH_2)_mOH \qquad (VI),$$

worin X Halogen, besonders Cl oder Br ist und m und n die zuvor angegebene Bedeutung haben, oder mit einem ungesättigten Alkohol der Formel VII

$$CH_2{=}CH(CH_2)_{m'-2}OH \qquad (VII),$$

worin m' für 3 bis 20 steht, erhalten werden.

Die Reaktion der Verbindungen der Formeln V und VI kann leicht durchgeführt werden, indem man stöchiometrische Mengen dieser Verbindungen in Gegenwart einer Base zur Entfernung des gebildeten Halogenwasserstoffs, und bei Temperaturen von 30 bis 120°C Umsetzt, und danach das gebildete Salz durch z.B. Waschen mit Wasser entfernt. Geeignete Basen sind z.B. Alkalimetall- und Erdalkalimetallhydroxide, Alkalimetallcarbonate, Carbamate und Amine, wie z.B. Trimethylamin oder Pyridin. Während der Reaktion sollte soviel Base Sugegeben werden, dass der gesamte gebildete Halogenwasserstoff gebunden wird. Die Reaktion kann in Gegenwart eines inerten organischen Lösungsmittels durchgeführt werden. Beispiele hierfür sind Toluol, Tetrahydrofuran, Dimethylsulfoxid und Alkanole mit vorzugsweise 1 bis 6 C-Atomen.

Die Additionsreaktion des Merkaptans der Formel V mit dem ungesättigten Alkohol der Formel VII wird vorteilhaft in Gegenwart eines Radikale bildenden Initiators bei Temperaturen von 30 bis 100°C durchgeführt. Geeignete Initiatoren sind z.B. Azoverbindungen wie 2,2'-Azobis-(2,4-dimethylvaleronitril). Die Reaktion kann in Gegenwart eines inerten organischen Lösungsmittels durchgeführt werden, z.B. Tetrahydrofuran, Methylethylketon oder Dimethylsulfoxid.

Die Perfluoralkylthiole der Formel V sind bekannt oder können nach bekannten Verfahren hergestellt werden, vergleiche die amerikanischen Patentschriften 3,655,732; 3,088,849; 3,145,222; 2,965,659; 2,972,638 und 3,544,663 und die australische Offenlegungsschrift 36 868.

Die erfindungsgemässen α,β-ungesättigten Ester sind sehr reaktiv und können zur Herstellung von Homo- und Copolymeren verwendet werden. Diese Polymeren sind ein weiterer Gegenstand der Erfindung.

Die Polymerisation der α,β-ungesättigten Ester erfolgt nach bekannten Methoden, wie sie zum Beispiel in Houben-Weyl, Methoden der Organischen Chemie, Band 14/1, S. 1044—1047, (Georg Thieme Verlag, Stuttgart, 1961) oder C. E. Schildknecht, Vinyl and Related Polymers, S. 179—255 (John Wiley and Sons Inc., New York 1952) beschrieben sind.

Die Polymerisation kann in Masse, Lösung, Suspension oder Emulsion vorgenommen werden. die Lösungs- und Emulsionspolymerisation sind bevorzugt.

Bei der Emulsionspolymerisation werden ein oder mehrere Monomere in der wässrigen Lösung eines oberflächenaktiven Mittels emulgiert. Die Konzentration an Monomeren beträgt im allgemeinen 5 bis 50 Gew.-%. Die Reaktionstemperatur kann 40 bis 70°C betragen. Die Polymerisation wird in Gegenwart eines Katalysators durchgeführt. Geeignete Katalysatoren sind alle Verbindungen, die zur Initiierung der Polymerisation von ethylenisch ungesättigten Verbindungen verwendet werden. Die Konzentration der Katalysatoren beträgt im allgemeinen 0,1 bis 2 Gew.-%, bezogen auf das Gewicht der Monomeren.

Die verwendeten oberflächenaktiven Mittel können kationischer, anionischer oder nichtionischer Art sein. Kationische und nichtionische Typen sind bevorzugt, da sie in den meisten Bädern zur Textilbehandlung verwendet werden. Der hydrophobe Teil des oberflächenaktiven Mittels kann ein Kohlenwasserstoffrest oder ein fluorierter Kohlenwasserstoffrest sein.

Geeignete nichtionische Oberflächenaktive Mittel sind z.B. solche, in denen die hydrophile Gruppe eine Polyethoxygruppe und der hydrophobe Teil entweder ein Kohlenwasserstoffrest oder ein pefluorierter Kohlenwasserstoffrest ist. Als Beispiele seien die Kondensate von Ethylenoxid mit Alkylphenolen, Alkanolen, Alkylaminen, Alkylthiolen, aliphatischen Carbonsäure, Fluoralkylcarbonsäuren oder Fluoralkylaminen, genannt.

Geeignete kationische oberflächenaktive Mittel sind z.B. quaternäre Ammoniumsalze oder Aminsalze, die mindestens eine langkettige Alkyl-, Fluoralkyl-, oder eine mit längerkettigem Alkyl substituierte Phenyl- oder Naphthylgruppe enthalten.

Die Polymerisationszeit hängt vom verwendeten Katalysator und der Temperatur und anderen Reaktionsbedingungen ab. Die Zeit beträgt im allgemeinen 0,5 bis 24 Stunden.

Die Polymerisationstemperatur hängt im wesentlichen vom verwendeten Katalysator ab. Bei der Emulsionspolymerisation im wässrigen Medium beträgt die Temperatur etwa 20 bis 90°C. Die Polymerisation wird, wenn möglich, bei Normaldruck durchgeführt.

Bei der Lösungspolymerisation wird mindestens ein Monomer in einem geeigneten Lösungsmittel gelöst, z.B. Tetrahydrofuran oder fluorierte Lösungsmittel wie z.B. Hexafluorxylol, Trifluortoluol oder Mischungen davon mit Aceton und/oder Ethylacetat. Die Polymerisation wird zweckmässig in Gegenwart eines Initiators, z.B. Azobisisobutyronitril oder anderen Azoinitiatoren, bei Temperaturen von 40 bis 100°C unter Stickstoff als Schutzgas durvhgeführt. Die Konzentration des Initiators beträgt bevorzugt 0,1 bis 2 Gew.-%, bezogen auf das Gewicht der Monomeren.

Die Monomeren der Formel I können homopolymerisiert oder mit üblichen Comonomeren, bevorzugt nichtfluorierten Comonomeren copolymerisiert werden. Die Comonomeren können hydrophil oder hydrophob oder Mischungen solcher Comonomeren sein. Um Schmutz ablösende Eigenschaften auf Textilien zu erzielen, werden vorteilhaft hydrophile Comonomere verwendet. Wenn hydrophobe und

oleophobe Eigenschaften gewünscht werden, verwendet man hauptsächlich hydrophobe Comonomere. Die Menge des Monomeren der Formel I im Copolymeren hängt hauptsächlich von der gewünschten Höhe der Oleophobie und gegebenenfalls Hydrophobie in der endgültigen Ausrüstung von textilen Materialien ab. Bevorzugt sind im Copolymer 10 bis weniger als 100, besonders 50 bis 99,9 und insbesondere 65 bis 90 Gew.-% Monomer der Formel I oder Mischungen davon enthalten, bezogen auf die Mischung der Comonomeren und des Monomer der Formel I.

Beispiele für Comonomere sind:

Ethylen, und Chloro-, Fluoro-, Amido- und Cyano-Derivate von Ethylen wie z.B. Vinylchlorid, Vinylidenchlorid, Vinylfluorid, Acrylnitril, Methacrylnitril, Acrylamid, Methacrylamid und N,N-Dimethylacrylamid, Tetrafluorethylen, Hexafluorpropylen; Acrylat- und Methacrylatmonomere, besonders solche mit 1 bis 18 C-Atomen in der Estergruppe wie z.B. n-Propylmethacrylat, 2-Methylchclohexylmethacrylat, Methylmethacrylat, t-Butylmethacrylat, n-Butylmethacrylat, Methylacrylat, Ethylacrylat, Propylacrylat, Butylacrylat, 3-Methylpentylacrylat, Octylacrylat, Tetradecylacrylat, s-Butylacrylat, 2-Ethylhexylacrylat, 2-Methoxyethylacrylat und Phenylacrylat; Diene, besonders 1,3-Butadien, Isopren, Chloropren, 2-Fluorbutadien, 1,1,3-Trifluorbutadien, 1,1,2,3-Tetrafluorbutadien, 1,1,2-Trifluor-3,4-dichlorbutadien und Tri- und Pentafluorbutadien and -isopren; N-Vinylmonomere wie z.B. Vinypyridin, N-Vinylpyridin, N-Vinylamid, N-Vinylsuccinimid, N-Vinylpyrrolidon, N-Vinylcarbazol; Styrol und Derivate von Styrol wie z.B. o-Methylstyrol, p-Methylstyrol, 3,4-Dimethylstyrol, m-Ethylstyrol, 2,5-Diethylstyrol; Vinylester und Vinylester von substituierten Säuren, wie z.B. Vinylmethoxyacetat, Vinyltrimethylacetat, Vinylisobutyrat, Isopropenbutyrat, Vinylacetat, Vinylcaprylat, Vinylpelargonat, Vinylmyristat, Vinyloleat und Vinyllinoleat; Vinylester von aromatischen Säuren wie z.B. Vinylbenzoat.

Lineare und verweigte α-Olefine mit 1 bis 10 C-Atomen in der Seitenkette sind vorteilhaft als Comonomere. Propylen, Butylen und Isobutylene sind von den α-Olefinen bevorzugt.

Vorteilhaft können auch Vinylcomonomere mit perfluorierten Seitenketten verwendet werden. Solche Comonomere sind z.B. in den US—Patentschriften 2,592,069 und 2,436,144 beschrieben. Weitere Comonomere sind Acrylate und Methacrylate und Derivate davon, die z.B. in den US—Patentschriften 2,628,958; 3,256,230; 2,839,513; 3,282,905; 3,252,932 und 3,304,278 beschrieben sind.

Es können auch geringe Mengen anderer reaktiver Comonomere mitverwendet werden, z.B. solche, mit denen die Wasch- und Trockenreinigungseigenschaften der Textilausrüstung gemäss der Erfindung verbessert werden kann. Solche Monomere reagieren während der Härtung als Vernetzungsmittel. Sie werden im allgemeinen in Mengen von 0,01 bis 5, bevorzugt 0,1 bis 2 Gew.-% verwendet, bezogen auf die Comonomeren.

Beispiele für reaktive Comonomere sind:

Acrylsäure, Methacrylsäure, Acrylamid, N-Methylolacrylamid, 2-Hydroxyethylmethacrylat oder -acrylat, Hydroxypropylacrylat oder -methacrylat, t-Butylaminoethylmethacrylat und Glycidylmethacrylat. N-Methylolacrylamid und 2-Hydroxyethylmethacrylat sind bevorzugt.

Ein weiterer Gegenstand vorliegender Erfindung ist ein Verfahren zur hydrophoben Ausrüstung von Cellulose und naturlichen oder synthetischen Polyamiden und Polyestern, bei dem auf besagten Materialien eine Schicht eines erfindungsgemässen Homo- oder Copolymer aufgebracht wird.

Beschichtungen mit Homo- und Copolymeren gemäss der Erfindung können mittels Lösungen oder wässrigen Emulsionen auf die Materialien aufgebracht werden. Geeignete Lösungsmittel sind Fluoralkane, Fluorchloralkane, Fluoralkyl substituierte Aromaten, Alkylester von Perfluoralkansäuren, Chloralkane und Chloraromaten, aromatische Kohlenwasserstoffe, Ketone, Ester und Ether.

Besonders vorteilhaft sind fluorierte Lösungsmittel, besonders α,α,α-Trifluortoluol (Benzotrifluorid), Hexafluorxylol und Mischungen davon mit Ethylacetat oder Aceton. Um eine ausreichende öl- und wasserabweisende Wirkung zu erzielen, beträgt die Konzentration der erfindungsgemässen Polymeren in der LÖsung im allgemeinen 0,01 bis 10 und bevorzugt 0,1 bis 2 Gew.-%, bezogen auf die Lösung, Mischungen von Emulsionen der erfindungsgemässen Polymeren mit Emulsionen von anderen Polymeren und Copolymeren sind besonders vorteilhaft für Textilausrüstungen. Die Polymeren und Copolymeren können Fluor enthalten. Bevorzugt sind Polymere und Copolymere, die kein Fluor enthalten. Beispiele sind Polymere von Alkylacrylaten und Alkylmethacrylaten, wie z.B. Methylmethacrylat, Ethylmethacrylat, Hexylmethacrylat, und n-Octylmethacrylat. Besonders bevorzugt ist Poly(n-octylmethacrylat). Weitere geeignete Polymere und Copolymere sind solche von Acrylsäure, Methacrylsäure, Styrol, Alkylstyrol, Butadien, 2-Methyl-1,3-butadien, 2-Chlor-1,3-butadien; Polymere und Copolymere von Vinylester wie z.B. Vinylacetat, Vinylbutyrat, Vinyllaurat, Vinylstearat, Vinyl-2-ethyl-hexanoat; Polymere und Copolymere von Vinylhalogenid und Vinylidenhalogeniden, wie z.B. Vinylchlorid, Vinylidenchlorid, Vinylfluorid, Vinylidenfluorid; Polymere und Copolymere von Allylestern wie z.B. Allylpropionat oder Allylcaprylat; Polymere und Copolymere von Vinylketonen, wie z.B. Methylvinylketon und Cetylvinylketon; Polymere und Copolymere von Acrylamiden, Methacrylamiden, N-Methylolacrylamid, N-Methylolmethacrylamid, N-Isopropylacrylamid und Methacrylnitrile.

Die erfindungsgemässen Homo- und Copolymeren weisen ölabstossende und Trockenschmutz resistente Eigenschaften auf. Die relative Bewertung der Eigenschaften erfolgt durch die Messung der Werte für die Oel- und Wasserabstossung und die Trockenschmutzresistenz. In den Beispielen werden die

folgenden Testverfahren verwendet:

Der AATCC-Oelwert wird nach dem Standardtestverfahren 118—1966 T der American Association of Textile Chemists and Colorists bestimmt. Die Werteskala reicht von 0 (Minimum) bis 8 (Maximum). Ein allgemein anerkannter Wert für die Schmutz abseisende Wirkung bei Textilien ist in USA ein Oelwert von 4.

Der Wert für den AATCC-Wassersprühtest wird gemäss dem Standardtestverfahren 22—1966 der American Association of Textile Chemists and Colorists XXVII, 1961, P. 1952 ermittelt (auch als ASTDM—D—583—58 bezeichnet). Die Werteskala reicht von 0 (Minumum) bis 100 (Maximum).

Der Widerstand gegen Trockenschmutz wird nach dem beschleunigten Bag-Verfahren bestimmt, das nachfolgend beschrieben wird:

Behandelte und unbehandelte Gewebe werden für eine bestimmte Zeit in einem Plastikbeutel in Gegenwart von Standard-Trockenschmutz geschüttelt. Ueberschüssiger Schmutz wird unter kontrollierten Bedeingungen entfernt und das beschmutzte Gewebe visuell beurteilt unter Benutzung der "Competitive Dry Soil Rating" Karte. Die Werte reichen von 0 (Minimum) bis 100 (Maximum).

Die Beschichtungen werden nach folgendem Verfahren auf das Gewebe aufgebracht. Ein Gewichtsprozent erfindungsgemässes Polymer wird in 1,1,1-Trichloroethan gelost. Die Lösung wird mittels eines Paddingverfahrens auf das Gewebe appliziert, danach an der Luft getrocknet und zusätzlich in einem Ofen bei 150°C während 5 Minuten gehärtet.

Die nachfolgenden Beilspeile erläutern die Erfindung.

Herstellung von Ausgangsverbindung

Beispiel A

Allylalkohol (44,4 g, 0,76 Mol) und $C_6F_{13}CH_2CH_2SH$ (190 g, 0,5 Mol) werden unter Stickstoff gemischt und die Lösung wird auf 55°C erwärmt. Azo-bis(2,4-dimethylvaleronitril), (1,4 g, 0,01 Mol) gelöst in Methylethylketon (15 Mol) wird während 50 Minuten zugegeben. Nach Beendigung der Zugabe wird die Lösung 1 Stunde bei 55°C gerührt und darauf 200 g Essigsäure zugefügt. Diese Lösung wird auf 70°C erwärmt, und 137 g 30%-ige Wasserstoffperixodlösung so zugegeben dass die Temperatur 85°C nicht übersteigt. Danach wird noch 45 Minuten bei 90°C gerührt und darauf gekühlt. Die Reaktionsmischung wird unter starkem Rühren in 2 Liter kaltes Wasser gegossen. Der gebildete weisse Niederschlag wird abfiltriert, mit 2%-iger Natronlauge und Wasser gewaschen und getrocknet. Man erhält 211 g (94% der Theorie) einer Verbindung der Formel $C_6F_{13}CH_2CH_2SO_2CH_2CH_2CH_2OH$, Schmelzpunkt 178—183°C.

$^1H$—NMR:
1,80 ppm, Multiplett, 2H, $CH_2\mathit{CH_2}CH\ OH$;
2,40—3,12 ppm, Multiplett, 2H, $C_6F_{13}\mathit{CH_2}CH$;
3,49 ppm, zwei Triplett, 4H, $\mathit{CH_2}SO_2\mathit{CH_2}$;
3,99 ppm, Triplett, 2H, $CH_2\mathit{CH_2}OH$.

Elementar-analyse:

| | | | | |
|---|---|---|---|---|
| Berechnet: | 28,1% C, | 2,3% H, | 52,6% F, | 6,8% S |
| Gefunden: | 28,2% C, | 2,5% H, | 52,8% F, | 6,8% S |

Beispiel B

Das Verfahren gemäss Beispiel A wird mit $C_8F_{17}CH_2CH_2SH$ wiederholt. Man erhält 91% der Theorie eines Alkohols der Formel $C_8F_{17}CH_2CH_2SO_2CH_2CH_2CH_2OH$, Schmelzpunkt 182—185°C.

$^1H$—NMR:
1,80 ppm, Multiplett, 2H, $\mathit{CH_2}CH_2CH_2OH$;
2,40—3,12 ppm, Multiplett, 2H, $C_6F_{13}\mathit{CH_2}CH$;
3,49 ppm, zwei Triplett, 4H, $\mathit{CH_2}SO_2\mathit{CH_2}$;
3,99 ppm, Triplett, 2H, $CH_2\mathit{CH_2}OH$.

Elementar-analyse:

| | | | |
|---|---|---|---|
| Berechnet: | 27,4% C, | 1,9% H, | 56,7% F |
| Gefunden: | 27,0% C, | 1,8% H, | 56,6% F |

Beispiel C

Beispiel A wird mit $R_fCH_2CH_2SH$ wiederholt. Man erhält 95% der Theorie eines Alkohols der Formel $R_fCH_2CH_2SO_2CH_2CH_2CH_2OH$, worin $R_f$ 6% $C_8F_{17}$, 64% $C_{10}F_{21}$, 25% $C_{12}F_{25}$, 5% $C_{14}F_{29}$ ist. Schmelzpunkt: 188—192°C.

$^1H$—NMR:

1,80 ppm, Multiplett, 2H, $\mathit{CH_2}CH_2CH_2OH$;
2,40—3,12 ppm, Multiplett, 2H, $C_6F_{13}\mathit{CH_2}CH$;
3,49 ppm, zwei Triplett, 4H, $\mathit{CH_2}SO_2\mathit{CH_2}$;
3,99 ppm, Triplett, 2H, $CH_2\mathit{CH_2}OH$.

Elementar-analyse:

| | | | | |
|---|---|---|---|---|
| Berechnet: | 26,1% C, | 1,6% H, | 58,0% F, | 4,7% S |
| Gefunden: | 26,1% C, | 1,5% H, | 59,4% F, | 4,6% S |

Herstellung von Estern

Beispiel 1

Triethylamin (21,1 g, 0,209 Mol) und $C_6F_{13}CH_2CH_2SO_2CH_2CH_2CH_2OH$ (89,0 g, 0,0189 Mol) werden in 1200 g trockenem Tetrahydrofuran gelöst und die Lösung bei Raumtemperatur unter Stickstoff gerührt. Man fügt tropfenweise 21,8 g (0,209 Mol) Methacroylchlorid zu und erhitzt über Nacht am Rückfluss. Es bildet sich ein Niederschlag.

Die Lösung wird filtriert und das Lösungsmittel verdampft. Der erhaltene Feststoff wird mit Methanol gewaschen. Man erhält 70% der Theorie eines Produktes der Formel

$$CH_2=\overset{\displaystyle CH_3 \atop |}{C}-CO_2CH_2CH_2CH_2SO_2CH_2CH_2C_6F_{13},$$ Schlemzpunkt 105,5—106°C.

$^1H$—NMR:

1,87 ppm, Singulett, 3H,

$$CH_2=\overset{\displaystyle \mathit{CH_3} \atop |}{C}-$$

1,90—2,75 ppm, Multiplett, 4H, $CH_2\mathit{CH_2}CH_2OH$, $C_6F_{13}\mathit{CH_2}CH_2$—;
3;37 ppm, Multiplett, 4H, $\mathit{CH_2}SO_2\mathit{CH_2}$;
4,10 ppm Triplett, 2H, $CH_2\mathit{CH_2}O$—;
5,65—6,02 ppm, Singuletts mit geringer Aufspaltung, 2H,

$$\mathit{CH_2}=\overset{\displaystyle CH_3 \atop |}{C}-.$$

Elementar-analyse:

| | | | |
|---|---|---|---|
| Berechnet: | 33,5% C, | 2,8% H, | 45,9% F |
| Gefunden: | 33,1% C, | 2,7% H, | 46,8% F |

Beispiel 2

Methylmethacrylat (350 g, 3,5 Mol), 2,6-di-t-butyl-4-methylphenol (2,4 g) und $C_8F_{17}CH_2CH_2SO_2CH_2CH_2CH_2OH$ (50,0 g, 0,088 Mol) werden unter Stickstoff vermischt. Die Lösung wird an einer 45 cm Kolonne, die mit einem Dean-Stark-Verschluss und Kühler ausgestattet ist, am Rückfluss erhitzt, um auch Spuren an Wasser zu entfernen. Als die Kopftemperatur 100°C erreicht, werden 60 ml azeotrope Mischung von Methylmethacrylat/Wasser entfernt. Es werden weitere 50 ml Methylmethacrylat zugegeben. Dann wird Tetraisopropyltitanat (9,0 g, 0,03 Mol) zugegeben und die Lösung unter Rückfluss erhitzt (Kopftemperatur 100°C), bis die Kopftemperatur un einige Grade fällt. Die Heizung wird entfernt, nachdem sich 32 ml Methylmethacrylat/Methanol-Azeotrop gebildet haben. Das überschüssige Methylmethacrylat wird unter Vakuum entfernt. Der Rückstand wird unter Rühren mit Tetrahydrofuran versetzt und der unlösliche Titanatkatalysator abfiltriert. Das Tetrahydrofuran wird dann im Vakuum entfernt. Man erhält 53,01 g (94% der Theorie) Produkt der Formel

$$C_8F_{17}CH_2CH_2SO_2CH_2CH_2CH_2O_2CC(CH_3)=CH_2.$$

$^1H$—NMR:

1,87 ppm, Singulett, 3H,

$$CH_2=\overset{\displaystyle \mathit{CH_3} \atop |}{C}-$$

1,90—2,75 ppm, Multiplett, 4H, $CH_2\mathit{CH_2}CH_2OH$, $C_6F_{13}\mathit{CH_2}CH_2$—;
3,37 ppm, Multiplett, 4H, $\mathit{CH_2}SO_2\mathit{CH_2}$;
4,18 ppm Triplett, 2H, $CH_2\mathit{CH_2}O$—;
5,65—6,02 ppm, Singuletts mit feiner Aufspaltung,

$$\begin{array}{r} CH_3 \\ | \\ \mathit{CH_2}{=}C- . \end{array}$$

Elementar-analyse:

| | | | | |
|---|---|---|---|---|
| Berechnet: | 32,0% C, | 2,4% H, | 50,6% F, | 5,0% S |
| Gefunden: | 31,8% C, | 2,1% H, | 50,7% F, | 5,4% S |

Beispiel 3

Das Verfahren gemäss Beispiel 1 wird mit dem Alkohol $R_fCH_2CH_2SO_2CH_2CH_2CH_2OH$ wiederholt. Man erhält 75% der Theorie eine Methacrylates der Formel

$$\begin{array}{r} CH_3 \\ | \\ R_fCH_2CH_2SO_2CH_2CH_2CH_2O_2C-C{=}CH_2, \end{array}$$

worin $R_f$ 6% $C_8F_{17}$, 64% $C_{20}F_{21}$, 25% $C_{12}F_{25}$, 5% $C_{14}F_{29}$ ist.

$^1H$—NMR:
1,80 ppm, Singulett, 3H,

$$\begin{array}{r} \mathit{CH_3} \\ | \\ CH_2{=}C- \end{array}$$

1,90—2,75 ppm, Multiplett, 4H, $CH_2\mathit{CH_2}CH_2OH$, $C_6F_{13}\mathit{CH_2}CH_2$—;
3,37 ppm, Multiplett, 4H, $\mathit{CH_2}SO_2\mathit{CH_2}$;
4,18 ppm Triplett, 2H, $CH_2\mathit{CH_2}O$—;
5,65—6,02 ppm, Singuletts mit feiner Aufspaltung, 2H,

$$\begin{array}{r} CH_3 \\ | \\ \mathit{CH_2}{=}C- . \end{array}$$

Elementar-analyse:

| | | | | |
|---|---|---|---|---|
| Berechnet: | 30,3% C, | 2,0% H, | 53,1% F, | 4,3% S |
| Gefunden: | 30,6% C, | 2,2% H, | 53,3% F, | 4,4% S |

Beispiele 4—12

Die Methacrylate gemäss Beispielen 1—3 werden zur Herstellung von Polymeren verwendet. Die Monomeren werden heiss in Tetrahydrofuran gelöst, Azo-bis(isobutyronitril) zugegeben und die Lösungen dann in evakuierten Ampullen luftdicht verschlossen. Die Polymerisation wird über Nacht bei 100°C in einem Rührbad durchgeführt. Die Zusammensetzung ergeben sich aus nachfolgender Tabelle.

| | Polymere von $CH_2{=}C(CH_3){-}CO_2(CH_2)_3SO_2(CH_2)_2R_f$ (A) und $CH_2{=}C(R){-}CO_2R_h$ (B) | | | | |
|---|---|---|---|---|---|
| Beispiel | $R_f$ | Gew.-% A | R | $R_h$ | Gew.-% B |
| 4 | $C_8F_{17}$ | 100 | - | - | - |
| 5 | * | 100 | - | - | - |
| 6 | $C_8C_{17}$ | 80 | $CH_3$ | $C_{13}H_{17}$ | 20 |
| 7 | * | 90 | $CH_3$ | $C_{13}H_{17}$ | 10 |
| 8 | * | 80 | $CH_3$ | $C_{13}H_{17}$ | 20 |
| 9 | * | 70 | $CH_3$ | $C_{13}H_{17}$ | 30 |
| 10 | $C_8F_{17}$ | 90 | H | 2 Ethylhexyl | 10 |
| 11 | $C_8F_{17}$ | 80 | H | 2 Ethylhexyl | 20 |
| 12 | $C_8F_{17}$ | 70 | H | 2 Ethylhexyl | 30 |

* $R_f$ ist eine Mischung von 6% $C_8F_{17}$, 64% $C_{10}F_{21}$, 25% $C_{12}F_{25}$ und 5% $C_{14}F_{29}$.

Beispiel 13

Die Polymeren der Beispiele 4, 6, 10, 11, und 12 werden auf 100%-Nylongewebe appliziert. Der Fluorgehalt beträgt 0,1 Gew.-%, bezogen auf das Gewebe. Die Ergebnisse sind in Tabelle 1 zusammengfasst.

| Polymer von Beispiel | Luft getrocknet | | | 5 Minuten bei 150°C im Ofen getrocknet | | |
|---|---|---|---|---|---|---|
| | $A^2$ Oelwert | $A^2$ Wassersprühtest | Trockenschmutzresistenz | $A^2$ Oelwert | $A^2$ Wassersprühtest | Trockenschmutzresistenz |
| 4 | 5 | 70 | 90 | 5-6 | 70 | 90 |
| 6 | 3 | 90 | 80 | 5 | 100 | 90 |
| 10 | 5 | 80 | 80-90 | 5 | 90 | 90-100 |
| 11 | 4-5 | 80 | 90 | 4-5 | 80 | 90-100 |
| 12 | 5 | 70 | 80 | 5-6 | 70 | 90 |

* $A^2$ bedeutet AATCC

**Patentansprüche**

1. Eine Perfluoralkylgruppe enthaltende Acrylate oder Methacrylate der Formel I

$$R_f(CH_2)_nSO_2(CH_2)_mO_2C{-}\overset{\displaystyle R}{\overset{|}{C}}{=}CH_2 \qquad (I),$$

worin $R_f$ für Perfluoralkyl oder Perfluoralkoxy substituiertes Perfluoralkyl mit bis zu 18 C-Atomen steht, n und m 2—20 sind und R Wasserstoff oder Methyl bedeutet.

2. Verbindungen gemäss Anspruch 1, worin R in Formel I Methyl ist.

3. Verbindungen gemäss Anspruch 1, worin in Formel I n und m 2—5 sind.

4. Verbindungen gemäss Anspruch 3, worin in Formel I n für 2 und m für 2—4 steht.

5. Verbindungen gemäss Anspruch 1, worin $R_f$ in Formel I 3 bis 18 C-Atome enthält.

6. Verbindungen gemäss Anspruch 1, worin in Formel I m für 3 steht.

7. Homopolymere der Verbindungen der Formel I gemäss Anspruch 1.

8. Copolymere von Verbindungen der Formel I gemäss Anspruch 1 mit mindestens einem Comonomer, wobei mindestens 10 und weniger als 100 Gewichtsprozent, bezogen auf das Copolymer, Monomer der Formel I enthalten sind.

9. Ein Copolymer gemäss Anspruch 8, worin das Monomer der Formel I in einer Menge von 50 bis 99,9 Gewichtsprozent enthalten ist.

10. Verfahren zur hydrophoben Ausrüstung von Cellulose und natürlichen oder synthetischen Polyamiden und Polyestern, dadurch gekennzeichnet, dass man aud fie Oberfläche dieser Materialien eine Schicht der Polymeren gemäss Ansprüchen 7 bis 8 aufbringt.

**Revendications**

1. Acrylates ou méthacrylates contenant un radical pefluoralkyle et répondant à la formule I:

$$R_f(CH_2)_nSO_2(CH_2)_mO_2C{-}\overset{\displaystyle R}{\overset{|}{C}}{=}CH_2 \qquad (I),$$

dans laquelle $R_f$ représente un radical perfluoroalkyle ou ou radical perfluoroalkyle éventuellement porteur d'un radical perfluoralcoxy et renfermant au plus 18 atomes de carbone, n et m représentent chacun un nombre de 2 à 20, et R représente l'hydrogène ou un méthyle.

2. Composé selon la revendication 1 dans lesquels le symbole R contenu dans la formule I représente un méthyle.

3. Composé selon la revendication 1 dans lesquels les indices n et m, dans la formule I, représentent chacun un nombre de 2 à 5.

4. Composé selon la revendication 3 caractérisés en ce que, dans la formule I, n est égal à 2 et m désigne un nombre de 2 à 4.

5. Composé selon la revendication 1 dans lesquels le symbole $R_b$ contenu dans la formule I renferme de 3 à 18 atomes de carbone.

6. Composé selon la revendication 1 caractérisés en ce que, dans la formule I, m est égal à 3.

7. Homopolymères des composés de formule I selon la revendication 1.

8. Copolymères de comosés de formule I selon la revendication 1 avec au moins un comonomère, la proportion du monomère de formule I, par rapport au copolymère, ètant d'au moins 10% e poids et inférieure à 100% en poids.

9. Copolymère selon la revendication 8 dans lequel le monomère de formule I est contenu en une quantité de 50 à 99,9% en poids.

10. Procédé pour rendre hydrophobesla cellulose ainsi que des polyesters et des polyamides naturels ou synthétiques, procédé caractérisé en ce qu'on applique à la surface de ces matières une couche des polymères selon l'une des revendciations 7 et 8.

**Claims**

1. A perfluoralkyl-containing acrylate or methacrylate of the formula I

$$R_f(CH_2)_nSO_2(CH_2)_mO_2C{-}\overset{\displaystyle R}{\overset{|}{C}}{=}CH_2 \qquad (I),$$

wherein

$R_f$ is perfluoroalkyl of up to 18 carbon atoms, or perfluoroalkoxy-substituted perfluoroalkyl of up to 18 carbon atoms, n and m are 2—20, and

R is hydrogen or methyl.

2. A compound according to claim 1, wherein R in formula I is methyl.

3. A compound according to claim 1, wherein in formula I n and m are 2—5.

4. A compound according to claim 3, wherein in formula I n is 2 and m is 2—4.

5. A compound according to claim 1, wherein $R_f$ in formula I contains from 3 to 18 carbon atoms.

6. A compound according to claim 1, wherein in formula I m is 3.

7. A homopolymer of the compound of the formula I according to claim 1.

8. A copolymer of the compound of the formula I according to claim 1 and at least one comonomer, wherein the monomer of the formula I is present in an amount of at least 10 and less than 100 per cent by weight, based on the copolymer.

9. A copolymer according to claim 8, wherein the monomer of the formula I is present in an amount of from 50 to 99.9 per cent by weight.

10. A method of imparting a hydrophobic finishing to cellulose and natural or synthetic polyamides or polyesters, comprising applying a coating of a polymer according to claim 7 or 8 onto the surface of these materials.